# EUROPEAN PATENT SPECIFICATION

(11) **EP 3 130 435 B1**
(45) Date of publication and mention of the grant of the patent: **03.06.2020**
(21) Application number: 15775994.5
(22) Date of filing: 09.04.2015
(51) Int. Cl.: A61K 8/73, A61Q 17/04, A61K 8/02, D04H 1/425, D21B 1/06, D21H 21/52

(54) **ANTI-ULTRAVIOLET SHEET-LIKE CELLULOSE MATERIAL AND PREPARATION METHOD AND USE THEREOF**
BLATTFÖRMIGES ANTI-ULTRAVIOLETT-CELLULOSEMATERIAL SOWIE HERSTELLUNGSVERFAHREN UND VERWENDUNG DAVON
MATÉRIAU DE CELLULOSE EN FORME DE FEUILLE ANTI-ULTRAVIOLETS ET SON PROCÉDÉ DE PRÉPARATION ET SON UTILISATION

(30) Priority: 10.04.2014 CN 201410143766
(43) Date of publication of application: 15.02.2017
(73) Proprietor: Technical Institute of Physics and Chemistry of the Chinese Academy of Sciences, Haidian District Beijing 100190 (CN)
(72) Inventor: WU, Min, Beijing 100190 (CN); HUANG, Yong, Beijing 100190 (CN); ZHAO, Mengmeng, Beijing 100190 (CN)
(74) Representative: MFG Patentanwälte Meyer-Wildhagen Meggle-Freund Gerhard PartG mbB
(86) International application number: PCT/CN2015/076157
(87) International publication number: WO 2015/154692

(56) References cited:
- EP-A1- 2 332 519
- WO-A1-2013/033833
- CN-A- 102 762 190
- CN-A- 103 211 719
- CN-A- 103 230 355
- CN-A- 103 442 685
- JP-A- 2004 230 719
- JP-A- 2014 028 785

## Description

### TECHNICAL FIELD

The present invention relates to the technical field of natural polymer materials, and more particularly relates to a flaky cellulose material with anti-ultraviolet function and a preparation method and a use thereof.

### BACKGROUND

In recent years, with the destruction of the ozone layer and the intensification of the sunspot activity, the ultraviolet dose radiated on the surface of the earth increases. Sunscreen products such as sunscreen cosmetics, sunscreen textiles and the like are more and more valued by people for avoiding damage caused by ultraviolet irradiation. The sunscreen effect of cosmetics is due to the absorption effect of the ultraviolet absorbent added in the cosmetics. However, with the role of sunscreen, the ultraviolet absorbent can screen also causes potential damage to human health such as skin injury, etc. For example, nano titanium dioxide known by people is currently the main additive of the sunscreen cosmetics. While, Titanium dioxide may also stimulate a free radical single-electron structure with ingredients of the sunscreen cosmetics under the irradiation of ultraviolet ray and visible light. This structure has a strong oxidizing ability and can cause damage to human skin cells.

Cellulose is the most abundant natural polymer in nature, which is mainly derived from plants. As an environment-friendly natural material, cellulose is non-toxic and harmless to human bodies, usually used as additive of food and drugs. The common morphology of cellulose materials is granular, fibrous, needlelike or rod-like. At present, there is no report on flaky cellulose nor its use as anti-ultraviolet material.

WO 2013/033833 A1 discloses topical compositions comprising nanocrystalline cellulose and at least one excipient exhibit enhanced photoprotection properties. They are therefore useful as sunscreens to protect against harmful ultraviolet (UV) radiation. Moreover, in some embodiments, the composition is a cream comprising at least about 2 % by weight of the nanocrystalline cellulose. The length, diameter, and aspect ratio of the crystals making up the nanocrystalline cellulose, as well as the crystallinity index (CRI) also preferably fall within select bounds.

CN 103 230 355 A discloses a sunscreen cream cosmetic composition containing nano crystal cellulose. The sunscreen cream cosmetic composition comprises the nano crystal cellulose and nano titanium dioxide. The sunscreen cream cosmetic composition is used for a sunscreen cream, is capable of preventing ultraviolet irradiation in a UVA (Ultraviolet A) region being 320-400nm and a UVB (Ultraviolet B) region being 280-320nm, and is high in sunscreen efficiency; and the sun protect factor (SPF) value is more than 15. The sunscreen cream is safe for a human body without toxicity, and can be suitable for being used by various kinds of persons.

EP 2 332 519 A1 discloses a powder cosmetic in which the powder component causes no aggregation and the surface powder component causes no caking in press processing even if a large amount of oily component is contained therein. Said powder cosmetic is characterized by comprising oblate cellulose particles and an oily component.

JP 2004 230719 A discloses flat cellulose particles which can be easily oriented and uniformly dispersed when added into another material and mixed with it. The flat cellulose particles have a width of 1-50 µm, a length of 1-50 µm and a thickness of 0.1-10 µm, and are obtained by mechanically grinding a cellulose substance. A mixture of a synthetic polymer, fatty acids, and water or an organic solvent is mechanically grounded to the cellulose substance.

### SUMMARY

The first technical problem to be solved in the present invention is to provide an anti-ultraviolet flaky cellulose material which can block transmission transmission of ultraviolet ray within a range of UVA (320-400 nm) and UVB (280-320 nm).

The second technical problem to be solved in the present invention is to provide a preparation method of the anti-ultraviolet flaky cellulose material.

The third technical problem to be solved in the present invention is to provide an application of the anti-ultraviolet flaky cellulose material.

According to a first aspect, the present invention provides an anti-ultraviolet flaky cellulose material according to independent claim 1.

According to a second aspect, the present invention provides a preparation method of an anti-ultraviolet flaky cellulose material according to independent claim 5.

According to a third aspect, the present invention provides a use of an anti-ultraviolet flaky cellulose material according to independent claim 9.

Further aspects of the invention are set forth in the dependent claims, the drawings and the following description.

To solve the above first technical problem, the present invention adopts the following technical solutions:
An anti-ultraviolet flaky cellulose material, which has a flaky morphology, with a particle width of 5-200 µm and a thickness of 0.01-10 µm. Because of special flaky structure, the cellulose material can block transmission of ultraviolet ray within the range of UVA (320-400 nm) and UVB (280-320 nm).

Preferably, the cellulose material is 30-50 µm in width and 0.01-10 µm in thickness. Preferably, the cellulose material is selected from natural cellulose or regenerated cellulose.

Preferably, the natural cellulose is selected from natural herbaceous plants or natural woody plants, and the regenerated cellulose is selected from one or more of viscose fiber, copper ammonia fiber, Tencel and alkali urea solution fiber.

To solve the above second technical problem, the present invention adopts the following technical solutions:
1) the cellulose material and a solid polymer abrasive material are mixed and milled; after milled, the morphology of the cellulose becomes flaky, thereby obtaining the flaky cellulose material; and
2) the flaky cellulose material obtained in step 1) is separated from the solid polymer abrasive material to obtain the anti-ultraviolet flaky cellulose material.

Preferably, the mixture in step 1) is the mixture of the solid polymer abrasive material and the cellulose material by weight ratio of 100-180:100, the milling is conducted in a ball mill or a grinding mill, the milling rate is 200-500 rpm, and the milling time is 2-30 h.

Preferably, the solid polymer abrasive material in step 1) is selected from polyolefin and a derivative and a copolymer thereof. Preferably, the solid polymer abrasive material is selected from one or more of polyethylene (PE), polypropylene (PP), polyvinyl chloride (PVC), polystyrene (PS), polyacrylonitrile, polyformaldehyde, epoxy resin, polyethylene terephthalate (PET), polymethyl methacrylate (PMMA), poly(butylene succinate) (PBS), polycarbonate (PC), poly(ε-caprolactone) (PCL), polylactic acid (PLA), polyamide, nylon 1010, polysulfone, bakelite, silicone rubber and fluoro alkylsilane (FAS).

Preferably, the cellulose material in step 1) is selected from natural cellulose or regenerated cellulose, the natural cellulose is selected from natural herbaceous plants or natural woody plants, and the regenerated cellulose is selected from one or more of viscose fiber, copper ammonia fiber, Tencel and alkali urea solution fiber.

Preferably, the separation of the flaky cellulose material from the solid polymer abrasive material in step 3) means separating the ground cellulose from the solid polymer abrasive material by using a sieve to make the width of the anti-ultraviolet flaky cellulose particle be 30-50 µm.

To solve the above third technical problem, the present invention adopts the following technical solutions:
The flaky cellulose material with an anti-ultraviolet function is applied to anti-ultraviolet cosmetics, anti-ultraviolet paint, anti-ultraviolet films, anti-ultraviolet fabric, etc.

### The present invention has the following beneficial effects:

In the prevent invention, cellulose powder and the solid polymer abrasive material are mechanically milled to obtain a new flaky cellulose material, which is not discovered previously. The flaky cellulose material has a function of blocking transmission of ultraviolet ray.

### BRIEF DESCRIPTION OF DRAWINGS

The specific embodiments of the present invention will be further described below in detail in conjunction with the accompanying figures.
Fig. 1 is SEM of the flaky cellulose material of embodiment 1.
Fig. 2 is UV-VIS reflection spectra of the flaky cellulose of embodiment 1.
Fig. 3 is UV-VIS reflection spectrum of flaky cellulose of embodiment 2, embodiment 6 and titanium dioxide nanoparticles.
Fig. 4 is UV-VIS reflection spectra of the flaky cellulose of embodiment 5.

### DETAILED DESCRIPTION

To describe the present invention more clearly, the present invention is further described below in combination with the preferred embodiments and the figures. Those skilled in the art should understand that the contents which are specifically described below are illustrative, rather than restrictive, and shall not be regarded as limiting the protection scope of the present invention.

### Embodiment 1

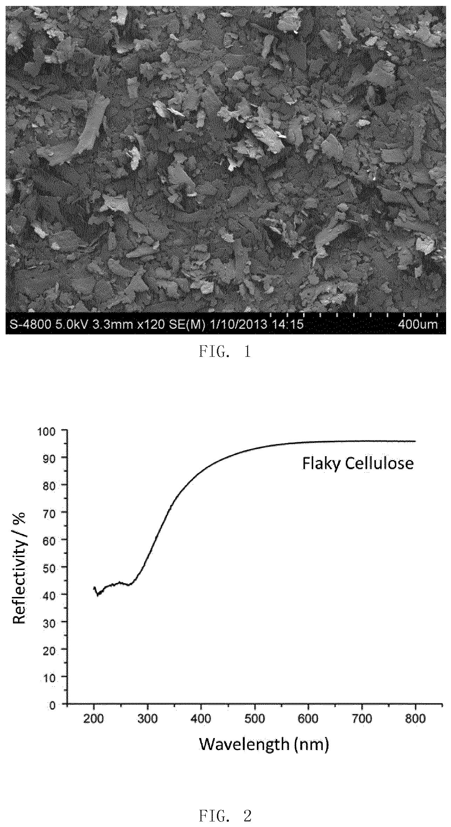

50 g of dry cellulose from a wood pulp is taken to be mixed with 50 g of polyethylene (PE) and mechanically milled for 16 hours. The milling balls are stainless steel balls with the diameter of 16 mm, and the grinding rate is 300 r/min. After milled , the cellulose powder and PE are separated by a stainless steel sieve to obtain the cellulose powder. The morphology of the obtained cellulose powder is flaky platelet, as shown in Fig. 1. The platelets are 40-80 µm wide 50-100 nm thick.

### Embodiment 2

50 g of dry cellulose from a wood pulp is taken to be mixed with 50 g of PE and mechanically milled for 4 h. The milling balls are stainless steel balls with the diameter of 16 mm, and the grinding rate is 500 r/min. After milled ground, the cellulose powder and PE are separated by a stainless steel sieve to obtain the cellulose powder. The morphology of the obtained cellulose powder is flaky platelet with the width of 80-200 µm and thickess of 1-5 µm.

### Embodiment 3

50 g of dry cellulose from a wood pulp is taken to be mixed with 50 g of polypropylene (PP) and mechanically milled for 28 h. The milling balls are stainless steel balls with the diameter of 16 mm, and the grinding rate is 500 r/min. After milled, the cellulose powder and PP are separated by a stainless steel sieve to obtain the cellulose powder. The morphology of the obtained cellulose powder is flaky platelet, with the width of 5-10 µm and thickness of 0.05-0.1 µm.

### Embodiment 4

30 g of dry cellulose from a cotton pulp is taken to be mixed with 50 g of PP and mechanically milled for 16 h. The grinding balls are stainless steel balls with the diameter of 16 mm, and the grinding rate is 300 r/min. After milled, the cellulose powder and PP are separated by a stainless steel sieve to obtain the cellulose powder. The morphology of the obtained cellulose powder is flaky platelet, with the width of 30-50 µm and thickness of 80-100 nm.

### Embodiment 5

20 g of dry copper ammonia regenerated cellulose is taken to be mixed with 30 g of polyvinyl chloride (PVC), and mechanically milld for 16 h. The grinding balls are a stainless steel balls with the diameter of 16 mm, and the grinding rate is 300 r/min. After milled, the cellulose powder and PVC are separated by a stainless steel sieve to obtain the cellulose powder. The morphology of the obtained cellulose powder is flaky platelet, with the width of 5-20 µm and thickness of 60-90 nm.

### Embodiment 6

30 g of dry microcrystalline cellulose is taken to be mixed with 50 g of PVC and mechanically milled for 16 h. The grinding balls are stainless steel balls with the diameter of 16 mm, and the grinding rate is 500 r/min. After milled, the cellulose powder and PVC are separated by a stainless steel sieve to obtain the cellulose powder. The morphology of the obtained cellulose powder is flaky platelet, with the width of 2-6 µm and thickness of 0.02-0.06 µm.

### Embodiment 7

The flaky cellulose obtained in embodiments 1-6 shows good anti-ultraviolet capability in UVA (320-400 nm) and UVB (280-320 nm). Fig. 2 is the UV-VIS reflection spectra of the flaky cellulose of embodiment 1. The flaky cellulose showsa reflectivity up to 85% against UVA and more than 50% against UVB, and almost total reflection against visible light. The 50 g of flaky cellulose is used for replacing common sunscreen additives such as nano titanium dioxide, octyl methoxycinnamate, benzophenone-3, etc. to be added to ingredients of the cosmetic ointment matrix formula: 100 g of white vaseline, 250 g of liquid paraffin, 1 g of mint, 50 g of dimethylcydosiloxane, 50 g of dimethyl siloxane, 100 g of glycerin, 2 g of sodium bisulfite, 2 g of sorbic acid, 140 g of emulsifier and 1000 g of distilled water. The content of the cellulose is 5%. The sun protection factor (SPF) value of the prepared cosmetic is higher than 30. The sunscreen cosmetic has sunscreen effect due to the reflection of the flaky cellulose against ultraviolet ray, which belongs to a mechanism of physical sunscreen, and has good capability of covering skin blemishes due to total reflection within the range of visible light. In addition, as a cosmetic additive, the flaky cellulose gives fine and smooth feel during use due to flaky morphology, better than other granular cosmetics. The sunscreen paint can be prepared by adding the flaky cellulose into the paint by the weight ratio of 10%, which can also be made into a film as a sunscreen film. Besides, the flaky cellulose can be added into the fabric to make sunscreen clothes and textiles.

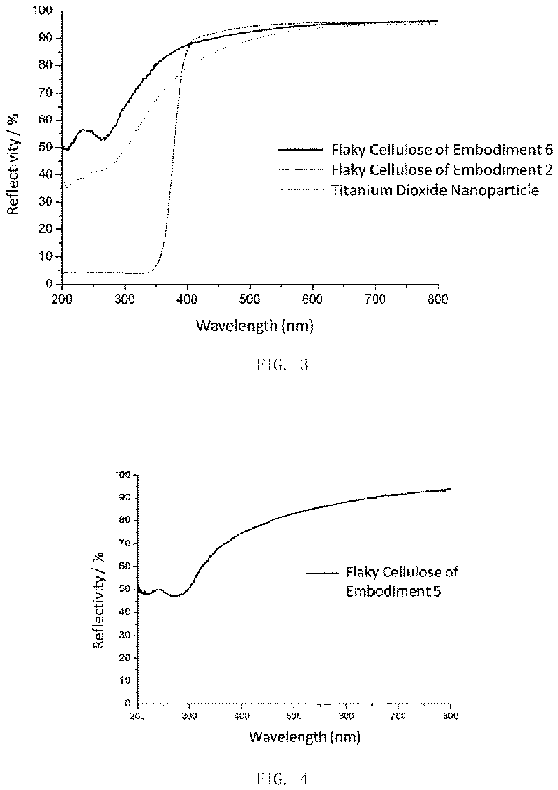

Fig. 3 is the reflection spectrum of flaky cellulose of embodiment 2, embodiment 6 and titanium dioxide nanoparticles. Fig. 4 is the reflection spectra of the flaky cellulose of embodiment 5. It can be seen from the above figures that although the size of the obtained flaky cellulose varies due to different raw materials of the cellulose, the reflection of the flaky cellulose against UVA, UVB and visible light is slightly different. The ultraviolet reflectivity of the flaky cellulose obtained in embodiments 1-6 is much higher than that of titanium dioxide nanoparticles, as shown in Fig. 3.

## Claims

1. An anti-ultraviolet flaky cellulose material for therapeutic use to block transmission of ultraviolet ray within a range of 320-400 nm and 280-320 nm, wherein the cellulose material has a flaky morphology, a particle width of 5-200 µm and a thickness of 0.01-10 µm.

2. The anti-ultraviolet flaky cellulose material according to claim 1, wherein the particle width of the cellulose material is 30-50 µm.

3. The anti-ultraviolet flaky cellulose material according to claim 1, wherein the cellulose is selected from natural cellulose or regenerated cellulose.

4. The anti-ultraviolet flaky cellulose material according to claim 3, wherein the natural cellulose is selected from natural herbaceous plants or natural woody plants, and the regenerated cellulose is selected from one or more of viscose fiber, copper ammonia fiber, and alkali urea solution fiber.

5. A preparation method of the anti-ultraviolet flaky cellulose according to claim 1, comprising the following steps:
1) mixing and milling the cellulose material and a solid polymer abrasive material, so that the morphology of the cellulose becomes flaky, thereby obtaining the flaky cellulose material; and
2) separating the flaky cellulose material obtained in step 1) from the solid polymer abrasive material to obtain the anti-ultraviolet flaky cellulose material; wherein
the separating the flaky cellulose material from the solid polymer abrasive material in step 2) is to separate the milled cellulose from the solid polymer abrasive material by using a sieve to make the particle width of the anti-ultraviolet flaky cellulose material be 30-50 µm.

6. The method according to claim 5, wherein the mixing in step 1) is to mix the solid polymer abrasive material and the cellulose material by the weight ratio of 100-180:100, and wherein the milling is conducted in a ball mill or a grinding mill, the milling rate is 200-500 rpm, and the milling time is 2-30 hours.

7. The method according to claim 5, wherein the solid polymer abrasive material in step 1) is selected from polyolefin and a derivative and a copolymer thereof; and preferably, the solid polymer abrasive material is selected from one or more of polyethylene, polypropylene, polyvinyl chloride, polystyrene, polyacrylonitrile, polyformaldehyde, epoxy resin, polyethylene terephthalate, polymethyl methacrylate, poly(butylene succinate), polycarbonate, poly(ε-caprolactone), polylactic acid, polyamide, nylon 1010, polysulfone, bakelite, silicone rubber and fluoro alkylsilane.

8. The method according to claim 5, wherein the cellulose material in step 1) is selected from natural cellulose or regenerated cellulose, the natural cellulose is selected from natural herbaceous plants or natural woody plants, and the regenerated cellulose is selected from one or more of viscose fiber, copper ammonia fiber, and alkali urea solution fiber.

9. A use of the anti-ultraviolet flaky cellulose material according to any one of claims 1-8 in an anti-ultraviolet paint, anti-ultraviolet films and anti-ultraviolet fabric.

## Patentansprüche

1. Anti-ultraviolettes blättchenartiges Zellulosematerial zur therapeutischen Anwendung zum Blockieren der Transmission von ultravioletter Strahlung in einem Bereich von 320-400 nm und 280-320 nm, wobei das Zellulosematerial eine blättchenartige Morphologie, eine Partikelbreite von 5-200 µm und eine Dicke von 0,01-10 µm aufweist.

2. Anti-ultraviolettes blättchenartiges Zellulosematerial nach Anspruch 1, wobei die Partikelbreite des Zellulosematerials 30-50 µm beträgt.

3. Anti-ultraviolettes blättchenartiges Zellulosematerial nach Anspruch 1, wobei die Zellulose aus natürlicher Zellulose oder Regenerat-Zellulose ausgewählt ist.

4. Anti-ultraviolettes blättchenartiges Zellulosematerial nach Anspruch 3, wobei die natürliche Zellulose aus natürlichen krautigen Pflanzen oder natürlichen Holzpflanzen ausgewählt ist und die Regenerat-Zellulose aus mindestens einem von Viskosefasern, Kupfer-Ammoniak-Fasern und Alkaliharnstofflösungs-Fasern ausgewählt ist.

5. Herstellungsverfahren für das anti-ultraviolette blättchenartige Zellulosematerial nach Anspruch 1, die folgenden Schritte umfassend:
1) Mischen und Mahlen des Zellulosematerials und eines festen Polymerschleifmaterials, sodass die Morphologie der Zellulose blättchenartig wird, wodurch das blättchenartige Zellulosematerial erhalten wird; und
2) Trennen des in Schritt 1) erhaltenen blättchenartigen Zellulosematerials vom festen Polymerschleifmaterial, um das anti-ultraviolette blättchenartige Zellulosematerial zu erhalten; wobei
das Trennen des blättchenartigen Zellulosematerials vom festen Polymerschleifmaterial in Schritt 2) zum Trennen der gemahlenen Zellulose vom festen Polymerschleifmaterial vorgesehen ist, wobei ein Sieb verwendet wird, um die Partikelbreite des anti-ultravioletten blättchenartigen Zellulosematerials von 30-50 µm herzustellen.

6. Verfahren nach Anspruch 5, wobei das Mischen in Schritt 1) zum Mischen des festen Polymerschleifmaterials und des Zellulosematerials im Gewichtsverhältnis von 100-180:100 vorgesehen ist, und wobei das Mahlen in einer Kugelmühle oder einer Mahlanlage durchgeführt wird, die Mahlrate 200-500 U/min beträgt und die Mahldauer 2-30 Stunden beträgt.

7. Verfahren nach Anspruch 5, wobei das feste Polymerschleifmaterial in Schritt 1) aus Polyolefin und einem Derivat und einem Copolymer davon gewählt ist; und wobei das feste Polymerschleifmaterial vorzugsweise aus einem oder mehreren der folgenden gewählt ist: Polyethylen, Polypropylen, Polyvinylchlorid, Polystyrol, Polyacrylnitril, Polyformaldehyd, Epoxidharz, Polyethylenterephthalat, Polymethylmethacrylat, Poly(butylensuccinat), Polycarbonat, Poly-(s-Caprolacton), Polymilchsäure, Polyamid, Nylon 1010, Polysulfon, Bakelit, Silikonkautschuk und Fluoralkylsilan.

8. Verfahren nach Anspruch 5, wobei das Zellulosematerial in Schritt 1) aus natürlicher Zellulose oder Regenerat-Zellulose gewählt ist, die natürliche Zellulose aus natürlichen krautigen Pflanzen oder natürlichen Holzpflanzen gewählt ist und die Regenerat-Zellulose aus einem oder mehreren der folgenden gewählt ist: Viskosefasern, Kupfer-Ammoniak-Fasern und Alkaliharnstofflösungs-Fasern.

9. Verwendung des anti-ultravioletten blättchenartigen Zellulosematerials nach einem der Ansprüche 1 bis 8 in einer anti-ultravioletten Farbe, anti-ultravioletten Film und anti-ultraviolettem Gewebe.

## Revendications

1. Matériau cellulosique floconneux anti-UV à usage thérapeutique pour arrêter la transmission de rayons ultraviolets dans une plage de 320-400 nm et 280-320 nm, dans lequel le matériau cellulosique a une structure floconneuse, une largeur de particule de 5-200 µm et une épaisseur de 0.01-10 µm.

2. Matériau cellulosique floconneux anti-UV selon la revendication 1, dans lequel la largeur de particule du matériau cellulosique est de 30-50 µm.

3. Matériau cellulosique floconneux anti-UV selon la revendication 1, dans lequel la cellulose est sélectionnée parmi la cellulose naturelle ou la cellulose régénérée.

4. Matériau cellulosique floconneux anti-UV selon la revendication 3, dans lequel la cellulose naturelle est sélectionnée parmi des plantes herbacées naturelles ou des plantes ligneuses naturelles, et la cellulose régénérée est sélectionnée parmi une ou plusieurs parmi les suivantes : fibre de viscose, fibre d'ammoniaque de cuivre et fibre de solution d'urée alcaline.

5. Procédé de préparation de cellulose floconneuse anti-UV selon la revendication 1, comprenant les étapes suivantes :
1) mélange et broyage du matériau cellulosique et d'un matériau polymère abrasif solide de sorte que la structure de la cellulose devienne floconneuse, obtenant ainsi le matériau cellulosique floconneux ; et
2) la séparation du matériau cellulosique floconneux obtenu dans l'étape 1) du matériau polymère abrasif solide pour obtenir le matériau cellulosique floconneux anti-UV ; dans lequel
la séparation du matériau cellulosique floconneux du matériau polymère abrasif solide dans l'étape 2) consiste à séparer la cellulose broyée du matériau polymère abrasif solide en utilisant un tamis pour que la largeur de particule du matériau cellulosique floconneux anti-UV soit de 30 à 50 µm.

6. Procédé selon la revendication 5, dans lequel le mélange à l'étape 1) consiste à mélanger le matériau polymère abrasif solide et le matériau cellulosique selon le rapport en poids de 100-180:100, et dans lequel le broyage est effectué dans un moulin à boulets ou un moulin broyeur, la vitesse de broyage est de 200-500 tours/minute, et le temps de broyage est de 2-30 heures.

7. Procédé selon la revendication 5, dans lequel le matériau polymère abrasif solide de l'étape 1) est sélectionné parmi une polyoléfine et un dérivé et un copolymère de celle-ci ; et de préférence, le matériau polymère abrasif solide est sélectionné parmi un ou plusieurs parmi polyéthylène, polypropylène, chlorure de polyvinyle, polystyrène, polyacrylonitrile, polyformaldéhyde, résine époxy, polytéréphtalate d'éthylène, poly-méthacrylate de méthyle, polybutylène succinate, polycarbonate, poly(ε-caprolactone), acide polylactique, polyamide, nylon 1010, polysulfone, bakélite, caoutchouc de silicone et fluoroalkylsilane.

8. Procédé selon la revendication 5, dans lequel le matériau cellulosique de l'étape 1) est sélectionné parmi la cellulose naturelle ou la cellulose régénérée, la cellulose naturelle est sélectionnée parmi les plantes herbacées naturelles ou les plantes ligneuses naturelles, et la cellulose régénérée est sélectionnée parmi une ou plusieurs de fibre de viscose, fibre d'ammoniaque de cuivre et fibre de solution d'urée alcaline.

9. Utilisation du matériau cellulosique floconneux anti-UV selon l'une des revendications 1-8 dans une peinture anti-UV, des films anti-UV et du tissu anti-UV.
